# EUROPÄISCHE PATENTANMELDUNG

(11) **EP 4 647 237 A2**
(43) Veröffentlichungstag der Anmeldung: **12.11.2025**
(21) Anmeldenummer: 25174998.2
(22) Anmeldetag: 08.05.2025
(51) Int. Cl.: B29C 49/42, B29C 49/06, B29C 49/46, B29L 31/00

(54) **VORRICHTUNG ZUM STERILISIEREN VON KUNSTSTOFFVORFORMLINGEN UND VERFAHREN HIERZU**

(30) Priorität: 08.05.2024 DE 102024112996
(71) Anmelder: Krones AG, 93073 Neutraubling (DE)
(72) Erfinder: Geltinger, Florian, 93073 Neutraubling (DE); Söllner, Jürgen, 93073 Neutraubling (DE)
(74) Vertreter: Bittner, Bernhard

(57) **Zusammenfassung**

Vorrichtung (1) zum Behandeln von Stückgütern (10) mit einer Transporteinrichtung (2), welche dazu geeignet und bestimmt ist, die Stückgüter zu transportieren und mit wenigstens einer Beaufschlagungseinrichtung (4), welche dazu geeignet und bestimmt ist, die von der Transporteinrichtung transportierten Stückgüter zu sterilisieren, insbesondere mit Strahlung zu sterilisieren und/oder mit einem fließfähigen Sterilisationsmittel zu beaufschlagen, wobei die Transporteinrichtung (2) eine erste bezüglich einer Drehachse (D) drehbare Transporteinheit (22) aufweist, welche eine Vielzahl von ersten Halteeinrichtungen (220, 222, 224) aufweist, welche jeweils zum Halten der Stückgüter geeignet und bestimmt sind und wobei die Transporteinrichtung (2) eine zweite bezüglich der Drehachse (D) drehbare Transporteinheit (24) aufweist, welche eine Vielzahl von zweiten Halteeinrichtungen (240, 242, 244) aufweist, welche jeweils in einer bezüglich der Drehachse (D) senkrecht stehenden zweiten Ebene (E2) angeordnet sind, dadurch gekennzeichnet, dass die Transporteinrichtung (2) wenigstens eine erste Hubeinrichtung (12) aufweist, welche dazu geeignet und bestimmt ist, einen vorgegebenen ersten Anteil der ersten Halteeinrichtungen (220) gegenüber einem vorgegebenen zweiten Anteil der ersten Halteeinrichtungen (222) in Richtung der Drehachse (D) zu verschieben.

## Beschreibung

Die vorliegende Erfindung bezieht sich auf eine Vorrichtung und ein Verfahren zum Behandeln und insbesondere Sterilisieren von Stückgütern und insbesondere von Kunststoffvorformlingen oder Behältnissen, insbesondere Kunststoffbehältnissen.

Aus dem Stand der Technik ist es seit langem bekannt, erwärmte Kunststoffvorformlinge zu Kunststoffbehältnissen umzuformen. Auch ist es im Stand der Technik seit langem bekannt, derartige umgeformte Kunststoffbehältnisse zu sterilisieren, beispielsweise wenn sie anschließend mit bestimmten Getränken befüllt werden sollen.

In jüngerer Zeit ist es jedoch auch bekannt geworden, die erwärmten Kunststoffvorformlinge zu sterilisieren und sie anschließend zu Behältnissen umzuformen, beispielsweise in einer Streckblasmaschine. Der Vorteil dieser Vorgehensweise besteht darin, dass die vergleichsweise kleinen Kunststoffvorformlinge und nicht deutlich größeren Behältnisse bzw. Flaschen sterilisiert werden müssen.

Zu diesem Zweck ist es bekannt, dass die Kunststoffvorformlinge in einen Behandlungsraum eingeführt werden und innerhalb dieses Behandlungsraums mit einem Sterilisationsmedium wie beispielsweise Wasserstoffperoxid beaufschlagt werden.

Diese Vorgehensweisen haben jedoch den Nachteil, dass sie in ihrer Effizienz begrenzt sind. Es wäre beispielsweise nicht möglich, die Kunststoffvorformlinge mit einer deutlich erhöhten Geschwindigkeit durch den Behandlungsraum zu transportieren, da dann die nötige Zeit für die Sterilisierung gegebenenfalls nicht ausreichend würde.

Die DE 10 2022 212 5620 A1 offenbart eine Behandlungsanlage mit einem Behandlungskarussell mit einer ersten und einer zweiten Behandlungsebene. Die Behältnisse werden in dem Behandlungskarussell auf einer ersten Ebene behandelt, anschließend von dem Karussell entnommen und dann auf eine zweite Ebene gefördert und auf dem Karussell auf dieser zweiten Ebene übergeben um dort wieder behandelt zu werden. Auf diese Weise können unterschiedliche Behandlungszeiten realisiert werden.

Die EP 0 998 418 B1 beschreibt eine mehrstöckige Vorrichtung zum Transport von Behältnissen. Diese Vorrichtung weist eine Vielzahl von Greiforganen auf, welche im Laufe einer Umdrehung der Vorrichtung vertikal verschoben werden können, um die Behältnisse auf verschiedene Ebenen anzuheben. Auf den einzelnen Ebenen kann eine Behandlung der Behältnisse erfolgen.

Der vorliegenden Erfindung liegt daher die Aufgabe zugrunde, eine Vorrichtung und ein Verfahren zur Verfügung zu stellen, welche die Effizienz und die Ausstoßleistung derartiger Sterilisationsanlagen verbessern. Dies wird erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Behandeln und insbesondere Sterilisieren von Stückgütern und insbesondere Kunststoffvorformlingen oder (Kunststoff)Behältnissen weist eine Transporteinrichtung auf, welche dazu geeignet und bestimmt ist, die Stückgüter entlang eines kreisförmigen Transportpfads zu transportieren. Daneben weist die Vorrichtung wenigstens eine Beaufschlagungseinrichtung auf, welche dazu geeignet und bestimmt ist, die von der Transporteinrichtung transportierten Stückgüter zu sterilisieren, insbesondere mit Strahlung zu sterilisieren und/oder mit einem fließfähigen Medium und insbesondere einem Sterilisationsmittel zu beaufschlagen.

Weiterhin weist die Transporteinrichtung eine erste bezüglich einer Drehachse drehbare Transporteinheit auf, welche Vielzahl von ersten Halteeinrichtungen aufweist, welche jeweils zum Halten der Stückgüter und insbesondere Kunststoffvorformlinge oder (Kunststoff)behältnisse geeignet und bestimmt sind. Dabei sind diese Halteeinrichtungen äquidistant (hintereinander) in einer bezüglich der Drehachse senkrecht verlaufenden ersten Ebene (und insbesondere einer bezüglich der Drehachse definierten Umfangsrichtung) angeordnet.

Weiterhin weist die Transporteinrichtung eine zweite bezüglich der Drehachse drehbare Transporteinheit auf, welche eine Vielzahl von zweiten Halteeinrichtungen aufweist, welche jeweils zum Halten der Stückgüter und insbesondere der Kunststoffvorformlinge oder (Kunststoff)behältnisse geeignet und bestimmt sind und welche äquidistant (hintereinander) in einer bezüglich der Drehachse senkrecht stehenden zweiten Ebene angeordnet sind, wobei die zweite Ebene in Richtung der Drehachse gegenüber der ersten Ebene versetzt ist.

Unter einer äquidistanten Anordnung der Halteeinrichtungen wird dabei insbesondere verstanden, dass diese eine konstante Teilung aufweisen, bzw. in der Umfangsrichtung jeweils gleiche Abstände zueinander haben.

Erfindungsgemäß weist die Transporteinrichtung wenigstens eine erste Hubeinrichtung auf, welche dazu geeignet und bestimmt ist, einen vorgegebenen ersten Anteil der ersten Halteeinrichtungen gegenüber einem vorgegebenen zweiten Anteil der ersten Halteeinrichtungen in Richtung der Drehachse zu verschieben.

Bevorzugt ist daher der besagte Hub auf der gleichen Transporteinrichtung durchführbar, welche auch die mehreren insbesondere übereinander angeordneten Transporteinheiten aufweist.

Es wird daher im Rahmen der Erfindung vorgeschlagen, dass ein bestimmter Anteil der ersten Halteeinrichtungen (insbesondere mit den daran angeordneten Kunststoffvorformlingen) in Richtung der Drehachse verschoben wird, also insbesondere angehoben oder abgesenkt wird. Auf diese Weise ist es, wie unten genauer erläutert möglich, mehrere Ebenen an Halteeinrichtungen mit Kunststoffvorformlingen oder allgemein Stückgütern zu besetzen, um auf diese Weise eine Möglichkeit zu schaffen, mehr Kunststoffvorformlinge insbesondere gleichzeitig zu sterilisieren oder allgemein zu behandeln.

Besonders bevorzugt ist damit die besagte erste Hubeinrichtung einem ersten Anteil der ersten Halteeinrichtungen zugeordnet. So ist es beispielsweise möglich, dass die erste Hubeinrichtung einen ersten Drittel der ersten Halteeinrichtungen zugeordnet ist.

Wie unten genauer beschrieben wird, erfolgt daher eine Behandlung und ein Transport der Stückgüter und insbesondere Kunststoffvorformlinge auf mindestens zwei Ebenen. Bevorzugt erfolgt eine Eingabe und Ausgabe, insbesondere über eine Halteeinrichtung (bei der es sich insbesondere um eine Neckhandlungsklammer handelt) auf einer Ebene. Die Stückgüter werden anschließend nach oben oder unten (d.h. entlang der Drehachse) bewegt.

Bevorzugt erfolgt generell die Verschiebung der Kunststoffvorformlinge oder allgemein Stückgüter, während diese in der Transportrichtung bzw. entlang des Transportpfads bewegt werden. Bevorzugt werden die Stückgüter kontinuierlich in der Transportrichtung bewegt und/oder sind kontinuierlich in der Transportrichtung bewegbar.

Bevorzugt werden die Stückgüter und insbesondere Kunststoffvorformlinge oder (Kunststoff)behältnisse erst frühestens bei jeder zweiten oder nachfolgenden Runde an eine weitere Transporteinrichtung wie einen Abführstern übergeben.

Bevorzugt werden die Stückgüter und insbesondere Kunststoffvorformlinge oder (Kunststoff)behältnisse in beiden Ebenen behandelt und insbesondere über Düsen mit gasförmigem Sterilisationsmittel begast. Bevorzugt erfolgt die Bewegung der Stückgüter und insbesondere Kunststoffvorformlinge oder (Kunststoff)behältnisse jeweils gruppenweise.

Die vertikale Bewegung bzw. die Hubbewegung in Richtung der Drehachse erfolgt bevorzugt elektrisch, pneumatisch oder kurvengesteuert.

Einer weiteren vorteilhaften Ausführungsform ist die Anzahl der ersten Halteeinrichtungen gleich der Anzahl der zweiten Halteeinrichtungen. Insbesondere ist daher die Anzahl der Halteeinrichtungen der zweiten Transporteinheit gleich der Anzahl der Halteeinrichtungen der ersten Transporteinheit.

Bevorzugt sind die Bewegungen der genannten Transporteinheiten in der Transportrichtung gekoppelt. Es bedeutet, dass sich hier die wenigstens zwei allgemein jedoch alle Transporteinheiten mit der gleichen Geschwindigkeit in der gleichen Drehrichtung bewegen. Bei einer bevorzugten Ausführungsform sind die Bewegungen aller Transporteinheiten in der Transporteinrichtung miteinander gekoppelt.

Besonders bevorzugt sind die zweiten Halteeinrichtungen (d.h. die Halteeinrichtungen der zweiten Transporteinheit) genau über oder unter den ersten Halteeinrichtungen (d.h. den Halteeinrichtungen der ersten Transporteinheit) angeordnet. Dies bedeutet beispielsweise dass ein Kunststoffvorformling, der von einer zweiten Halteeinrichtung gehalten wird geometrisch direkt oberhalb oder unterhalb einem Kunststoffvorformling ist, der von einer der ersten Halteeinrichtungen gehalten wird. Dabei ist jedoch bevorzugt zwischen diesen beiden Kunststoffvorformlingen wenigstens eine Beaufschlagungseinrichtung (insbesondere zur Beaufschlagung des unteren Kunststoffvorformlings) vorgesehen.

Dabei bedeutet unmittelbar bzw. direkt übereinander angeordnet, dass die geometrischen Längsachsen oder Drehachse dieser beiden Kunststoffvorformlinge zusammenfallen. Mit anderen Worten sind in einer Projektion in Richtung der Drehachse der Transporteinrichtung sämtliche zweiten Halteeinrichtungen (der zweiten Transporteinheit) genau oberhalb oder unterhalb den jeweiligen ersten Halteeinrichtungen (der ersten Transporteinheit) angeordnet.

Besonders bevorzugt handelt es sich bei den Stückgütern um Kunststoffvorformlinge oder Kunststoffbehältnisse. Besonders bevorzugt sind die zweiten Halteeinrichtungen jeweils in der Längsrichtung der Kunststoffvorformlinge genau über den ersten Halteeinrichtungen angeordnet.

Besonders bevorzugt sind die oben erwähnte erste Ebene und die zweite Ebene wenigstens um die Länge eines Stückguts und insbesondere eines Kunststoffvorformlings voneinander beanstandet. Besonders bevorzugt sind die Ebenen weiter voneinander beabstandet, als es der Längsausdehnung der Kunststoffvorformlinge entspricht. Besonders bevorzugt sind jedoch die Ebenen weniger als die dreifache Länge und bevorzugt weniger als die doppelte Länge der Stückgüter insbesondere weniger als die dreifache Länge und insbesondere weniger als die doppelte Länge der Kunststoffvorformlinge voneinander beanstandet. Durch diese Abmessungen kann einerseits eine effektive Sterilisation der Kunststoffvorformlinge erreicht werden und andererseits der Bauraum für die Vorrichtung noch gering gehalten werden.

Besonders bevorzugt handelt es sich bei den Halteeinrichtungen um Greifeinrichtungen und insbesondere Greifklammern. Besonders bevorzugt sind diese Halteeinrichtungen bzw. Greifklammern dazu geeignet und bestimmt, die Kunststoffvorformlinge in einem Mündungsbereich und insbesondere unterhalb deren Tragringe zu halten. Besonders bevorzugt handelt es sich bei den Greifklammern um steuerbare Greifklammern.

Bevorzugt ist daher wenigstens eine der Transporteinheiten segmentiert ausgebildet, wobei jedem Segment eine der Vielzahlen von Halteeinrichtungen zugeordnet sind. Bevorzugt sind mehrere und besonders bevorzugt alle Transporteinheiten in dieser Weise segmentiert ausgebildet.

Bevorzugt sind wenigstens drei solcher Segmente vorgesehen. Auf diese Weise können zwei der Segmente während der Beladung mit Kunststoffvorformlingen und während der Entladung der Kunststoffvorformlinge auf einer konstanten Höhe bleiben und das dritte Segment oder weitere Segmente können in der Höhe bzw. entlang der Drehachse verschoben werden

Bei einer weiteren Vorderreifen Ausführungsform weist die Vorrichtung ein Gehäuse auf, innerhalb dessen ein Behandlungsraum zum Behandeln und insbesondere Sterilisieren der Stückgüter und insbesondere der Kunststoffvorformlinge ausgebildet ist. Besonders bevorzugt ist die oben erwähnte Transporteinrichtung innerhalb dieses Behandlungsraums angeordnet. Bevorzugt handelt es sich bei diesem Behandlungsraum um einen Reinraum, der gegenüber einer (unsterilen) Umgebung abgeschirmt ist.

Besonders bevorzugt bewirkt dieses Gehäuse dabei einen luftdichten oder im wesentlichen luftdichten Abschluss des Behandlungsraums. Unter im Wesentlichen wird dabei verstanden, dass eventuell vorhandene Schleusen, durch welche Kunststoffvorformlinge zugeführt oder abgeführt werden außer Acht gelassen werden.

Bei einer weiteren vorteilhaften Ausführungsform sind die Halteeinrichtungen und insbesondere Greifklammern aus einem Material hergestellt, welches resistent gegen Sterilisationsmedien ist. Besonders bevorzugt sind die Halteeinrichtungen in der Transportrichtung äquidistant zueinander angeordnet. Besonders bevorzugt ist der erste Anteil der ersten Halteeinrichtungen gleich dem zweiten Anteil der ersten Halteeinrichtungen.

Besonders bevorzugt ist die Gesamtzahl der Halteeinrichtungen jeder Transporteinheit durch drei teilbar.

Bei einer bevorzugten Ausführungsform ist die erste Hubeinrichtung dazu geeignet und bestimmt, einen vorgegebenen ersten Anteil der zweiten Halteeinrichtungen gegenüber einem vorgegebenen zweiten Anteil der zweiten Halteeinrichtungen in Richtung der Drehachse zu verschieben.

Dies bedeutet, dass nicht die Gesamtheit der ersten Halteeinrichtungen notwendigerweise verschoben wird, sondern lediglich ein bestimmter Anteil. Besonders bevorzugt sind diese Halteeinrichtungen in der Umfangsrichtung der Transporteinrichtung hintereinander angeordnet. Auf diese Weise wird bevorzugt ein Segment mit einer Vielzahl von Halteeinrichtungen verschoben oder ist entsprechend verschiebbar.

Bevorzugt ist diese Verschiebung des vorgegebenen ersten Anteils der zweiten Halteeinrichtungen an die Verschiebung des vorgegebenen ersten Anteils der ersten Halteeinrichtungen gekoppelt.

Dies bedeutet, dass bevorzugt ein erstes Segment mit dem ersten Anteil der ersten Halteeinrichtungen und ein entsprechendes Segment mit einem ersten Anteil der zweiten Halteeinrichtungen entlang der Drehachse verschoben wird, wobei bevorzugt diese Segmente in Richtung der Drehachse übereinander liegen. Besonders bevorzugt sind die Anteile der ersten Halteeinrichtungen, die verschoben werden und die Anteile der zweiten Halteeinrichtungen, die verschoben werden, identisch bzw. gleich groß.

Bei dem Anteil der ersten Halteeinrichtungen könnte es sich auch um einzelne Halteeinrichtungen handeln. Bevorzugt handelt es sich jedoch um mehrere Halteeinrichtungen, bevorzugt um wenigstens zwei, bevorzugt wenigstens drei, bevorzugt, um wenigstens vier, bevorzugt wenigstens sechs und bevorzugt um wenigstens zehn Halteeinrichtungen.

Bevorzugt handelt es sich jedoch um Gruppen von in der Transporteinrichtung unmittelbar hintereinander angeordneten Halteeinrichtungen.

Auf diese Weise ist es möglich, die einzelnen Transporteinheiten nacheinander mit Kunststoffvorformlingen oder allgemein mit Stückgütern zu bestücken bzw. zu beladen.

Bei einer weiteren Vorderreifen Ausführungsform weist die Transporteinrichtung einen Zuführbereich auf, in welchem der Transporteinrichtung Stückgüter und insbesondere Kunststoffvorformlinge zuführbar sind, wobei die Stückgüter in einer Zuführebene zuführbar sind, bezüglich derer die Drehachse senkrecht steht.

Weiterhin weist die Transporteinrichtung bevorzugt einen Abführbereich auf, in welchem die Stückgüter und insbesondere Kunststoffvorformlinge von der Transporteinrichtung abführbar sind, wobei die Stückgüter in einer Abführebene zuführbar sind, bezüglich derer die Drehachse senkrecht steht.

Bei einer bevorzugten Ausführungsform liegen die Zuführebene und die Abführebene, auf der im wesentlichen gleichen Ebene bzw. auf der im wesentlichen gleichen Höhe bezüglich der Drehachse. Unter im Wesentlichen der gleichen Ebene wird hierbei verstanden, dass die Höhenniveaus dieser Ebenen sich um nicht mehr als 5cm, bevorzugt um nicht mehr als 3cm und bevorzugt um nicht mehr als 1cm und bevorzugt um nicht mehr als 0,5cm unterscheiden.

Bevorzugt verbleiben die Zuführebene und die Abführebene konstant, d.h. die Stückgüter werden stets in der gleichen Ebene zugeführt und abgeführt.

Besonders bevorzugt fallen daher die Zuführebene und die Abführebene im wesentlichen zusammen, wobei der Zuführbereich gegenüber dem Abführbereich in der Umfangsrichtung der ersten Transporteinrichtung versetzt ist.

Bei einer weiteren vorteilhaften Ausführungsform weist die Transporteinrichtung eine dritte bezüglich der Drehachse drehbare Transporteinheit auf, welche eine Vielzahl von dritten Halteeinrichtungen aufweist, welche jeweils zum Halten der Stückgüter und insbesondere Kunststoffvorformlinge oder Behältnisse geeignet und bestimmt sind und welche bevorzugt äquidistant in einer bezüglich der Drehachse senkrecht stehenden dritten Ebene angeordnet sind. Dabei ist diese dritte Ebene in der Richtung der Drehachse gegenüber der ersten Ebene und der zweiten Ebene versetzt.

Bei einer bevorzugten Ausführungsform ist ein Abstand zwischen der dritten Ebene und der zweiten Ebene gleich dem Abstand zwischen der zweiten Ebene und der ersten Ebene. Bei einer weiteren vorteilhaften Ausführungsform ist die Anzahl der dritten Halteeinrichtungen gleich der Anzahl der ersten Halteeinrichtungen und der Anzahl der zweiten Halteeinrichtungen.

Bevorzugt liegen auch die dritten Halteeinrichtungen genau über den ersten und/oder zweiten Halteeinrichtungen.

Besonders bevorzugt entsprechen die Transportkreise der einzelnen Halteeinrichtungen der ersten und zweiten und bevorzugt auch der dritten Transporteinheit einander.

Es wird darauf hingewiesen, dass die hier beschrieben Vorrichtung zum Behandeln der Stückgüter auch zwei oder mehrere der hier beschriebenen Transporteinrichtungen aufweisen kann. Bevorzugt sind wenigstens zwei und bevorzugt wenigstens drei derartige Transporteinrichtungen vorgesehen. Bevorzugt sind alle diese Transporteinrichtungen in dem oben beschriebenen Behandlungsraum angeordnet.

Bei einer weiteren bevorzugten Ausführungsform kann die Transporteinrichtung auch mehr als drei Transporteinheiten aufweisen.

Bei einer weiteren bevorzugten Ausführungsform weist die Transporteinrichtung eine zweite Hubeinrichtung auf, welche dazu geeignet und bestimmt ist, den vorgegebenen zweiten Anteil der ersten Halteeinrichtungen gegenüber dem vorgegebenen ersten Anteil der ersten Halteeinrichtungen in Richtung der Drehachse zu verschieben.

Bei der Hubeinrichtung könnte es sich beispielsweise auch um eine Führungskurve handeln, welche mit mehreren Kurvenrollen zusammenwirkt, welche einzelne Segmente heben und senken können. In diesem Falle wird unter einer Hubeinrichtung jeweils die Kombination dieser Führungskurve mit einer bestimmten Kurvenrolle und dem entsprechenden Träger der Kurvenrolle verstanden, der dann die jeweiligen Halteeinrichtungen heben und senken kann. Daher wäre auch bei einer Ausgestaltung mit einer zweiten Führungsrolle eine zweite Hubeinrichtung vorgesehen. Bevorzugt weisen die Hubeinrichtungen auch jeweils Führungseinrichtungen auf, welche zum Führen der Verschiebebewegung entlang der Drehachse geeignet und bestimmt sind.

Bei einer weiteren bevorzugten Ausführungsform weist die Transporteinrichtung eine dritte Hubeinrichtung auf, welche dazu geeignet und bestimmt ist, einen vorgegebenen dritten Anteil der ersten Halteeinrichtungen gegenüber dem vorgegebenen ersten Anteil der ersten Halteeinrichtungen und/oder gegenüber dem vorgegebenen ersten Anteil der zweiten Halteeinrichtungen in Richtung der Drehachse zu verschieben. Auch bei dieser dritten Hubeinrichtung kann es sich wieder um eine elektrisch, pneumatisch, hydraulisch oder durch eine Führungskurve betätigte Hubeinrichtung handeln.

Bei einer weiteren bevorzugten Ausführungsform sind mehr als drei bzw. n Hubeinrichtungen vorgesehen.

Bei einer bevorzugten Ausführungsform ist daher eine erste Transporteinheit mit ersten Halteeinrichtungen, eine zweite Transporteinheit mit zweiten Halteeinrichtungen und eine dritte Transporteinheit mit dritten Halteeinrichtungen vorgesehen. Diese ersten, zweiten und dritten Halteeinrichtungen weisen bevorzugt jeweils erste, zweite und dritte Anteile an Halteeinrichtungen auf.

Besonders bevorzugt ist daher die erste Hubeinheit dem ersten Anteil der ersten Halteeinrichtungen zugeordnet, die zweite Hubeinheit dem zweiten Anteil der ersten Halteeinrichtungen und die dritte Hubeinheit einem dritten Anteil der ersten Halteeinrichtungen. Bevorzugt sind diese ersten, zweiten und dritten Anteile jeweils gleich groß.

Auf diese Weise ist es im laufenden Betrieb stets möglich, beispielsweise über einen entlang der Drehachse konstant gehaltenen ersten Anteil der Halteeinrichtungen oder ein erstes Segment Kunststoffvorformlinge zuzuführen und über ein weiteres entsprechend entlang der Drehachse konstant gehaltenes beispielsweise drittes Segment Kunststoffvorformlinge abzuführen.

Ein zwischen diesen beiden ersten und dritten genannten Segmenten liegendes Segment kann hingegen verschoben werden, um auf diese Weise die vollständige Beladung der Transporteinrichtung, insbesondere mit Kunststoffvorformlinge oder Behältnissen zu ermöglichen.

Bei einer weiteren bevorzugten Ausführungsform ist die zweite Hubeinrichtung dazu geeignet und bestimmt, einen vorgegebenen zweiten Anteil der zweiten Halteeinrichtungen gegenüber einem vorgegebenen ersten Anteil der zweiten Halteeinrichtungen in Richtung der Drehachse zu verschieben.

Besonders bevorzugt ist diese Verschiebung des vorgegebenen zweiten Anteils der zweiten Halteeinrichtungen an die Verschiebung von vorgegebenen zweiten Anteils der ersten Halteeinrichtungen gekoppelt.

Insgesamt kann man sich bildlich die Zusammenstellung aus den zwei oder bevorzugt drei Transporteinheiten derart vorstellen, dass einzelne Segmente etwa in der Art von Kuchenstücken jeweils gegenüber verbleibenden Segmenten angehoben oder abgesenkt werden können.

Bei einer weiteren bevorzugten Ausführungsform weist die Beaufschlagungseinrichtung eine Vielzahl von Beaufschlagungselementen und insbesondere Beaufschlagungsdüsen auf, welche bevorzugt jeweils den Halteeinrichtungen zugeordnet sind und welche besonders bevorzugt dazu geeignet und bestimmt sind, die Innenoberflächen der Kunststoffvorformlinge mit einem Sterilisationsmedium zu sterilisieren und/oder beaufschlagen.

Bevorzugt weist die Vorrichtung eine Vielzahl von Beaufschlagungssegmenten auf, welche jeweils gemeinsam mit den jeweiligen Anteilen von Halteeinrichtungen bezüglich der Drehachse verschiebbar sind. Besonders bevorzugt sind jeder Transporteinheit jeweils mehrere derartige Beaufschlagungssegmente zugeordnet, wobei bevorzugt jedem Anteil der jeweiligen Halteeinrichtungen einer jeden Transporteinheit wenigstens ein derartiges Beaufschlagungssegment zugeordnet sind. Bevorzugt sind die Beaufschlagungssegmente gleichartig bzw. als Gleichteile ausgebildet.

Bevorzugt ist daher wenigstens eine Einrichtung der Beaufschlagungseinrichtung (und insbesondere wenigstens eines der oben genannten Beaufschlagungssegmente) entlang der Drehachse verschiebbar. Bevorzugt ist dabei die Bewegung dieser Einrichtung, d.h. insbesondere des Beaufschlagungssegments an die Bewegung wenigstens eines Anteils der Halteeinrichtungen gekoppelt.

Bei einer weiteren bevorzugten Ausführungsform sind bevorzugt diese Beauschlagungseinrichtungen und/oder Beaufschlagungssegmente oberhalb der ihnen zugeordneten Halteeinrichtungen angeordnet. Auf diese Weise wird eine Beaufschlagung der mit den Mündungen nach oben transportierten Kunststoffvorformlinge ermöglicht.

Besonders bevorzugt ist die Transporteinrichtung in einem insbesondere abgeschlossenen Gehäuse angeordnet.

Besonders bevorzugt weist die Transporteinrichtung und/oder die Vorrichtung eine (zentrale) Versorgungseinheit für die Beaufschlagung der Kunststoffvorformlinge mit einem Sterilisationsmedium auf. Besonders bevorzugt handelt es sich bei dem Sterilisationsmedium um Wasserstoffperoxid oder Peressigsäure.

Ebenso bevorzugt weist die Transporteinrichtung und/oder die Vorrichtung eine (zentrale) Strahlungserzeugungsvorrichtung, insbesondere Strahlungsquelle, zur Sterilisation der Kunststoffvorformlinge mit Strahlung, auf. Besonders bevorzugt handelt es sich bei der Strahlung um Elektronenstrahlung und/oder ultravioletter Strahlung.

Bei einer weiteren bevorzugten Ausführungsform weist die Vorrichtung einen Drehverteiler auf. Dieser Drehverteiler dient dazu, um von einer stationären Quelle des Sterilisationsmediums dieses auf die jeweiligen Träger und insbesondere die einzelnen Beaufschlagungseinrichtungen zu verteilen wobei diese Übertragung insbesondere auch während einer Bewegung der Transporteinheiten möglich sein soll.

Bei einer weiteren bevorzugten Ausführungsform weist die Vorrichtung einen Zuführstern oder ein Zuführrad auf, welches die Kunststoffvorformlinge der Transporteinrichtung zuführt.

Bei einer weiteren bevorzugten Ausführungsform weist die Vorrichtung einen Abführstern oder ein Abführrad auf, welches die Kunststoffvorformlinge von der Transporteinrichtung abführt. Besonders bevorzugt ist die Transporteinrichtung gemeinsam mit dem Zuführstern und dem Abführstern in einem Gehäuse angeordnet.

Bei einer weiteren bevorzugten Ausführungsform weist die Vorrichtung eine Vielzahl von sich entlang einer kreissegmentförmigen Linie erstreckenden Trägersegmenten auf, welche jeweils zum gemeinsamen Tragen einer Vielzahl von Halteeinrichtungen geeignet und bestimmt sind. Bevorzugt ist dabei jedes der Trägersegmente zum Tragen eines der Anteile der Halteeinrichtungen, die oben bezeichnet wurden, geeignet und bestimmt.

Bei einer weiter bevorzugten Ausführungsform wird die Strahlung von wenigstens einer Strahlungserzeugungsvorrichtung, insbesondere Strahlungsquelle, als Elektronenstrahlung und/oder ultravioletter Strahlung bereitgestellt und/oder die Strahlung von der Beaufschlagungseinrichtung über ein, bevorzugt eine Vielzahl von, Beaufschlagungselementen insbesondere einen Strahlfinger und/oder einen Flächenstrahler aufgebracht.

Bevorzugt enthält die Vorrichtung wenigstens eine, bevorzugt mehrere, Strahlungszeugungsvorrichtung, insbesondere einen Elektronenstrahler und/oder einen ultravioletten (UV) Strahler.

Bevorzugt ist die Beaufschlagungseinrichtung dazu geeignet, Strahlung auf die Kunststoffvorformlinge aufzubringen und sie dadurch zu sterilisieren.

Die Sterilisation der Kunststoffvorformlinge erfolgt bevorzugt mittels Elektronenstrahlen. Die Beaufschlagungseinrichtung weist dabei wenigstens ein Beaufschlagungselement, insbesondere einen, bevorzugt mehrere, Flächenstrahler auf, der bevorzugt entlang des Transportpfades der Kunststoffvorformlinge angeordnet ist und bevorzugt auf deren Außenflächen gerichtet ist. Der wenigstens eine Flächenstrahler ist bevorzugt, relativ zu der Transporteinrichtung stationär angeordnet, bevorzugt ist dieser für die Sterilisation, insbesondere der Kunststoffvorformlinge, von außen geeignet.

Die Beaufschlagungseinrichtung weist dabei wenigstens ein Beaufschlagungselement, insbesondere einen Strahlfinger, bevorzugt mehrere Strahlfinger, auf, welche in die Kunststoffvorformlinge einführbar sind, und bevorzugt in die Kunststoffvorformlinge eingeführt werden.

Bevorzugt ist der Strahlfinger, bevorzugt sind die Vielzahl der Strahlfinger, mit der Transporteinrichtung mitbewegbar, und bevorzugt werden sie mit der Transporteinrichtung und/oder den Kunststoffvorformlingen mitbewegt. Bevorzugt sind die Strahlfinger zur Innensterilisation geeignet und/oder vorgesehen, Dafür können sie bevorzugt in ein Inneres der Kunststoffvorformlinge eintauchen.

Die Sterilisation erfolgt bevorzugt in einem Teilschritt.

Als Strahlfinger wird ein beliebig geformter, bevorzugt in eine Behältnisöffnung, insbesondere Kunststoffvorformlingsöffnung, einführbarer, stangenartiger Körper verstanden.

Bevorzugt wird bei der Sterilisation der Kunststoffvorformlinge durch Strahlung die Außenseite und/oder Innenseite sterilisiert/ entkeimt. Dabei dreht sich der Kunststoffvorformling vor/um einer Strahlungserzeugungsvorrichtung um seine Längsachse, sodass alle Oberflächenpunkte der Außenseite und/oder Innenseite des Kunststoffvorformlings eine möglichst gleichmäßige Bestrahlung in Qualität und Quantität erfahren.

Um den Kunststoffvorformlinge um seine Längsachse zu drehen, wird vorgeschlagen, einen Rotationsdorn, bevorzugt mehrere Rotationsdorne zu integrieren, auf die der Kunststoffvorformling aufsteckbar ist und insbesondere von unten aufgesteckt wird. Die Dorne werden wenigstens im Bereich der Elektronenquelle um ihre Längsachse rotiert, um die Außenhülle des Kunststoffvorformlings möglichst gleichmäßig zu bestrahlen.

Die Beaufschlagungseinrichtung weist bevorzugt als Beaufschlagungselemente eine Vielzahl von Strahlfingern auf, welche in die Kunststoffvorformlinge einführbar sind, um diese mit Strahlung, insbesondere Elektronenstrahlung und/oder ultraviolette Strahlung zu beaufschlagen.

Besonders bevorzugt sind jeweils Elektronenbeschleunigungseinrichtungen vorgesehen und/oder auch Austrittsfenster zum Austritt der Elektronenstrahlen, die bevorzugt aus Titan ausgebildet sind.

Bei einer weiteren bevorzugten Ausführungsform ist die Sterilisation als eine Außen- und/oder Innensterilisation, insbesondere auf derselben Transporteinrichtung, ausführbar und/oder eine Außen- und/oder Innensterilisation auf wenigstens zwei, bevorzugt einer Vielzahl von, unterschiedlichen Transporteinrichtungen, insbesondere zeitlich und/oder örtlich nach-/hintereinander, ausführbar.

Bevorzugt behandelt, insbesondere sterilisiert, die Beaufschlagungseinrichtung eine Außenoberfläche der Kunststoffvorformlinge.

Besonders bevorzugt wird zu diesem Zweck ein Flächenstrahler vorgesehen, der Strahlung und insbesondere Elektronenstrahlung und/oder ultraviolette Strahlung auf die Außenoberfläche der Kunststoffvorformlinge richtet. Dabei kann dieser Flächenstrahler derart an einem Gehäuse, insbesondere der Vorrichtung, angeordnet sein, dass die Strahlung durch eine Öffnung des Gehäuses hindurch auf die Kunststoffvorformlinge trifft.

Bevorzugt behandelt, insbesondere sterilisiert, die Beaufschlagungseinrichtung eine Innenoberfläche der Kunststoffvorformlinge.

Besonders bevorzugt wird zu diesem Zweck ein Strahlfinger vorgesehen, der Strahlung und insbesondere Elektronenstrahlung und/oder ultraviolette Strahlung auf die Innenoberfläche der Kunststoffvorformlinge richtet. Dabei kann dieser Strahlfinger derart an einem Gehäuse, insbesondere der Vorrichtung, angeordnet sein, dass die Strahlung durch eine Öffnung des Gehäuses hindurch auf die Kunststoffvorformlinge trifft. Dabei können beispielsweise Strahlungszeugungsvorrichtungen, insbesondere Elektronenstrahler und/oder ultraviolette (UV) Strahler, in die Behältnisse eingeführt werden.

Besonders bevorzugt finden sowohl eine Außen- als auch eine Innenoberflächen Sterilisierung der Kunststoffvorformlinge statt.

Die Beaufschlagungseinrichtung mit den Beaufschlagungselementen zur Außen- und/oder Innensterilisierung kann dabei gemeinschaftlich, oder getrennt voneinander ausgeführt sein.

Bevorzugt ist die Außen- und/oder die Innensterilisation an einem Kunststoffvorformling zeitlich und örtlich gleich ausführbar.

Besonders bevorzugt ist die Außen- und/oder die Innensterilisation an einem Kunststoffvorformling zeitlich nacheinander ausführbar.

Besonders bevorzugt ist die Außen- und/oder die Innensterilisation an einem Kunststoffvorformling örtlich hintereinander ausführbar.

Dabei ist denkbar die Außen- und/oder die Innensterilisation an unterschiedlichen Transporteinrichtungen und/oder Halteeinrichtungen durchzuführen.

Bevorzugt erfolgt die Beaufschlagung der Kunststoffvorformlinge mit einem fließfähigen Medium zeitlich vor der Behandlung der Außenoberfläche und/oder der Innenoberfläche der Kunststoffvorformlinge mit Strahlung. Insbesondere wird der Kunststoffvorformling bevorzugt zuerst mit einem fließfähigen Medium vorgereinigt und anschließend mit Elektronen- und/oder ultravioletter Strahlung sterilisiert/entkeimt.

Hierdurch können besonders gute Sterilisierungs-/Entkeimungsergebnisse erzielt werden.

Besonders bevorzugt trägt jedes Trägersegment wenigstens drei bevorzugt wenigstens vier, bevorzugt wenigstens fünf, bevorzugt wenigstens sechs, bevorzugt wenigstens acht und bevorzugt wenigstens zehn Halteeinrichtungen. Besonders bevorzugt sind dabei diese Halteeinrichtungen einzeln von diesem Trägersegment demontierbar und/oder einzeln an diesem Trägersegment montierbar.

Die vorliegende Erfindung ist weiterhin auf eine Transporteinrichtung zum Transportieren von Kunststoffvorformlingen oder (Kunststoff)Behältnissen entlang eines kreisförmigen Transportfahrt gerichtet, wobei die Transporteinrichtung eine erste bezüglich einer Drehachse drehbare Transporteinheit aufweist, welche eine Vielzahl von ersten Halteeinrichtungen aufweist, welche jeweils zum Halten der Kunststoffvorformlinge oder Behältnisse geeignet und bestimmt sind und welche äquidistant in einer bezüglich der Drehachse senkrecht stehenden ersten Ebene angeordnet sind.

Weiterhin weist die Transporteinrichtung eine zweite bezüglich der Drehachse drehbare Transporteinheit auf, welche eine Vielzahl von zweiten Halteeinrichtungen aufweist, welche jeweils zum Halten der Kunststoffvorformlinge oder Kunststoffbehältnissen geeignet und bestimmt sind und welche äquidistant in einer bezüglich der Drehachse senkrecht stehenden zweiten Ebene angeordnet sind, wobei die zweite Ebene in der Richtung der Drehachse gegenüber der ersten Ebene versetzt ist und wobei ein Versatz der zweiten Ebene gegenüber der ersten Ebene in Richtung der Drehachse größer ist als eine Länge der zu transportierenden Kunststoffvorformlinge oder Behältnisse in dieser Richtung der Drehachse.

Erfindungsgemäß weist die Transporteinrichtung wenigstens eine erste Hubeinrichtung auf, welche dazu geeignet und bestimmt ist, einen vorgegebenen ersten Anteil der ersten Halteeinrichtungen gegenüber einem vorgegebenen zweiten Anteil der ersten Halteeinrichtungen in Richtung der Drehachse zu verschieben und weiterhin weist die Transporteinrichtung eine zweite Hubeinrichtung auf, welche dazu geeignet und bestimmt ist, den vorgegebenen zweiten Anteil der ersten Halteeinrichtungen gegenüber dem vorgegebenen ersten Anteil der ersten Halteeinrichtungen in Richtung der Drehachse zu verschieben.

Besonders bevorzugt ist die Transporteinrichtung in der oben beschriebenen Weise ausgeführt. Dies bedeutet, dass die oben beschriebenen Merkmale in Bezug auf die Transporteinrichtung der Vorrichtung ebenso für die hier beschriebene Transporteinrichtung Anwendung finden können.

Bevorzugt ist diese Transporteinrichtung dazu geeignet, die Kunststoffvorformling und/oder Behältnisse während einer vorgegebenen Behandlung, insbesondere einer Sterilisation zu transportieren. Es wäre allerdings auch möglich, dass es sich bei dieser Behandlung um einen Erwärmungsvorgang, einen Spülvorgang, einen Inspektionsvorgang der Kunststoffvorformling oder dergleichen handelt.

Besonders bevorzugt weisen die beiden Hubeinrichtungen elektrische Antriebe auf. Es wären jedoch auch pneumatische oder hydraulische Antriebe oder Antriebe mittels Führungskurven denkbar.

Die vorliegende Erfindung ist weiterhin auf ein Verfahren zum Behandeln und insbesondere Sterilisieren von Stückgütern und insbesondere Kunststoffvorformlingen oder (Kunststoff)Behältnissen gerichtet, wobei eine Transporteinrichtung die Stückgüter und insbesondere Kunststoffvorformlinge oder (Kunststoff)Behältnisse entlang eines kreisförmigen Transportpfads transportiert und wenigstens eine Beaufschlagungseinrichtung die von der Transporteinrichtung transportierten Stückgüter und insbesondere Kunststoffvorformlinge oder Kunststoffbehältnisse mit einem fließfähigen Sterilisationsmittel und/oder sterilisierender Strahlung beaufschlagt.

Weiterhin weist die Transporteinrichtung eine erste bezüglich einer Drehachse drehbare Transporteinheit auf, welche eine Vielzahl von ersten Halteeinrichtungen aufweist (bzw. an der diese Halteeinrichtungen angeordnet sind), welche jeweils die Stückgüter und insbesondere Kunststoffvorformlinge oder (Kunststoff)Behältnisse halten und welche äquidistant in einer bezüglich der Drehachse senkrecht stehenden ersten Ebene (insbesondere in der Umfangsrichtung) angeordnet sind.

Weiterhin weist die Transporteinrichtung eine zweite bezüglich der Drehachse drehbare und/oder drehende Transporteinheit auf, welche eine Vielzahl von zweiten Halteeinrichtungen aufweist, welche jeweils die Stückgüter und insbesondere die Kunststoffvorformlinge oder (Kunststoff)behältnisse halten und welche äquidistant in einer bezüglich der Drehachse senkrecht stehenden zweiten Ebene angeordnet sind (und insbesondere einer Umfangsrichtung, welche in dieser Ebene verläuft), wobei die zweite Ebene in der Richtung der Drehachse gegenüber der ersten Ebene versetzt ist. Insbesondere sind diese beiden Ebenen parallel zueinander.

Erfindungsgemäß weist die Transporteinrichtung wenigstens eine erste Hubeinheit auf, welche wenigstens zeitweise einen vorgegebenen ersten Anteil der ersten Halteeinrichtungen gegenüber einem vorgegebenen zweiten Anteil der ersten Halteeinrichtungen in Richtung der Drehachse verschiebt.

Besonders bevorzugt ist eine Transportzeit für alle Stückgüter mittels der Transporteinrichtung gleich. Dies ist von Bedeutung, da auf diese Weise auch die Einwirkzeit bzw. die Sterilisationszeit für alle Kunststoffvorformlinge gleich gehalten wird.

Besonders bevorzugt sind oder werden die Drehungen der Transporteinheiten miteinander gekoppelt.

Bei einer weiteren bevorzugten Ausführungsform werden die Stückgüter und insbesondere die Kunststoffvorformlinge und/oder Behältnisse der Transporteinrichtung in einer Zufuhrebene zugeführt, zu welcher die Drehachse senkrecht steht.

Weiterhin werden die Stückgüter und insbesondere die Kunststoffvorformlinge oder Kunststoffbehältnisse von der Transporteinrichtung in einer Abführebene abgeführt. Dabei steht die Drehachse auch senkrecht zu dieser Abführebene. Besonders bevorzugt fallen die Zufuhrebene und die Abwehrebene (im Wesentlichen) zusammen. Dies bedeutet, dass die Stückgüter und insbesondere Kunststoffvorformlinge oder Behältnisse in der gleichen Ebene oder der gleichen Höhe zugeführt werden, in der sie auch wieder abgeführt werden.

Bei einem weiteren bevorzugten Verfahren wird jeder Kunststoffvorformling und/oder jedes Kunststoffbehältnis um einen Transportwinkel transportiert, der größer ist als 360°, bevorzugt größer als 720°. Damit werden bevorzugt Stückgüter und insbesondere die Kunststoffvorformlinge oder Behältnisse um mehr als 1 Umdrehung und bevorzugt um mehr als 2 Umdrehungen transportiert.

Bei einer weiteren bevorzugten Ausführungsform wird jedes Stückgut und insbesondere jeder Kunststoffvorformling oder jedes Behältnis wenigstens einmal in Richtung der Drehachse in einer ersten Verschieberichtung verschoben und wenigstens einmal in Richtung der Drehachse in einer zweiten Verschieberichtung verschoben, welche der ersten Verschieberichtung entgegengesetzt ist.

Bei einem weiteren bevorzugten Verfahren wird jedes Stückgut und insbesondere jeder Kunststoffvorformling und/oder jedes Behältnis wenigstens zweimal in Richtung der Drehachse in einer ersten Verschieberichtung verschoben. Bevorzugt wird auf diese Weise ein Beladen der Transporteinrichtung mit den Stückgütern und insbesondere den Kunststoffvorformlingen oder Behältnissen durchgeführt.

Besonders bevorzugt erfolgt diese genannte Verschiebung auch in der zweiten Richtung, welche der ersten Richtung entgegengesetzt ist, wenigstens einmal bevorzugt wenigstens zweimal.

Bei einem weiteren bevorzugten Verfahren werden Anteile von in der Transport Richtung hintereinander angeordneten Kunststoffvorformlingen und/oder Kunststoffbehältnissen gemeinsam entlang der Drehachse verschoben. Bei einem weiteren bevorzugten Verfahren werden übereinander angeordnete Kunststoffvorformlinge oder Behältnisse gemeinsam entlang der Drehachse verschoben.

Bevorzugt wird also während einer Produktion bzw. während eines Behandlungsvorgang jeweils wenigstens eines der Segmente entlang der Drehachse verschoben. Bevorzugt wird zu einem vorgegebenen Zeitpunkt oder Zeitraum jedoch nur genau eines der Segmente entlang der Drehachse verschoben.

Bei einer weiteren bevorzugten Ausführungsform weist die Behandlungseinrichtung wenigstens eine Transporteinrichtung auf, welche dazu geeignet und bestimmt ist, einen Abstand aufeinanderfolgender Kunststoffvorformlinge zu verändern (auch als Teilungsverzugsstern bezeichnet). Bei einer weiteren bevorzugten Ausführungsform weist die Behandlungseinrichtung wenigstens zwei Transporteinrichtungen auf, welche dazu geeignet und bestimmt sind, einen Abstand aufeinanderfolgender Kunststoffvorformlinge zu verändern.

Bevorzugt ist die Behandlungseinrichtung daher in mehrere Gruppen von dem Halteeinrichtungen zugeordnete Behandlungselementen wie insbesondere Behandlungsdüsen unterteilt. Diese können dabei sowohl an den unterschiedlichen Transporteinheiten als auch in unterschiedlichen Segmenten angeordnet sein. Bevorzugt liegen dabei wenigstens vier Gruppen, bevorzugt wenigstens sechs Gruppen und besonders bevorzugt wenigstens neun Gruppen vor (insbesondere je Transporteinheit wenigstens drei Gruppen).

Bei einer weiteren bevorzugten Ausführungsform ist eine Steuerungseinrichtung vorgesehen, welche dazu geeignet ist, einzelne Transporteinheiten zu aktivieren oder zu deaktivieren. Es wäre also möglich, Stückgüter und insbesondere Kunststoffvorformlinge nur einer der genannten Transporteinheiten oder nur zwei den genannten Transporteinheiten zuzuführen. Auf diese Weise kann die Speicherkapazität der Transporteinheit verändert werden.

Bei einer weiteren bevorzugten Ausführungsform weist die Transporteinrichtung eine Dichtungseinrichtung auf, welche die Transporteinrichtung gegenüber eine (unsterilen) Umgebung abdichtet. Hierbei kann es sich beispielsweise um eine hydraulische Dichtung handeln. Es könnten beispielsweise sog. Wasserschlösser zur Abdichtung eingesetzt werden. Derartige Wasserschlösser weisen bevorzugt einen umlaufenden mit Wasser oder einer anderen Flüssigkeit gefüllten stationären Kanal auf, in welchen ein umlaufendes Schwert eintaucht um so sie Abdichtung zu bewirken.

Bei einer weiteren bevorzugten Ausführungsform ist die Transporteinrichtung innerhalb deines Reinraums angeordnet.

Bevorzugt weist die Vorrichtung wenigstens eine Sensoreinrichtung auf, welche dazu geeignet und bestimmt ist, wenigstens eine Eigenschaft zu erfassen, welche für ein innerhalb des Behandlungsraums befindliches gasförmiges Medium charakteristisch ist. Bei dieser Eigenschaft kann es sich beispielsweise um einen Druck, eine Temperatur oder eine Konzentration an Sterilisationsmittel handeln.

Weitere Vorteile und Ausführungsformen ergeben sich aus den beigefügten Figuren.

Darin zeigen:
- Fig. 1: eine schematische Darstellung einer Anlage zum Herstellen von Kunststoffbehältnissen;
- Fig. 2: eine Darstellung einer erfindungsgemäßen Transporteinrichtung; und
- Fig. 3: eine Darstellung zur Veranschaulichung eines erfindungsgemäßen Verfahrens.

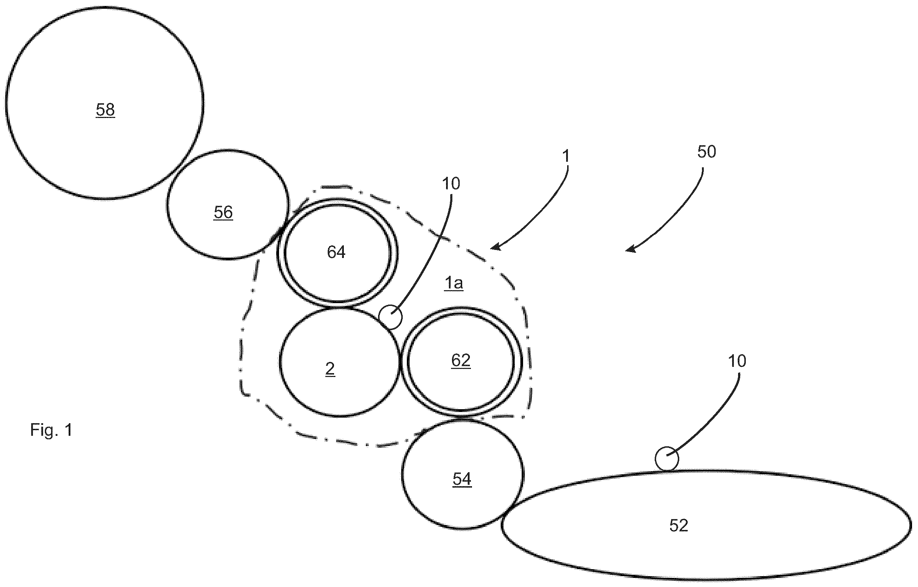

Fig. 1 zeigt eine schematisch eine Anlage 50 zum Herstellen von Kunststoffbehältnissen. Diese Anlage weist eine Erwärmungseinrichtung 52 auf, welche die Kunststoffvorformlinge 10 erwärmt. Hierbei kann es sich beispielsweise um einen Infrarotofen oder einen Mikrowellenofen handeln. Das Bezugszeichen 54 kennzeichnet eine Transporteinrichtung, welche die erwärmten Kunststoffvorformlinge der Behandlungseinrichtung 1, bei der es sich um eine Sterilisationseinrichtung handelt, zuführt. Bevorzugt handelt es sich bei dieser Transporteinrichtung 54 um einen sog. Teilungsverzugsstern, der einen Abstand bzw. eine Teilung zwischen den Kunststoffvorformlingen ändern kann.

Das Bezugszeichen 1 kennzeichnet die Behandlungseinrichtung. Das Bezugszeichen 1a kennzeichnet einen Behandlungsraum, innerhalb dessen die Behandlung und insbesondere Sterilisation der Kunststoffvorformlinge vorgenommen wird. Das Bezugszeichen 2 kennzeichnet die oben beschriebene Transporteinrichtung, der von einer Zuführeinrichtung 62 Kunststoffvorformlinge zugeführt werden und von der mittels einer Abführeinrichtung 64 die sterilisierten Kunststoffvorformlinge abgeführt werden.

Das Bezugszeichen 56 kennzeichnet eine weitere Transporteinrichtung, bevorzugt wieder in Form eines Teilungsverzugssterns, welche die sterilisierten Kunststoffvorformlinge abführt, Bevorzugt ist diese weitere Transporteinrichtung 56 daher dazu geeignet und bestimmt, eine Teilung zwischen den transportierten Kunststoffvorformlinge zu verändern und insbesondere zu vergrößern.

Das Bezugszeichen 58 kennzeichnet eine Umformungseinrichtung wie insbesondere eine Blasformmaschine und insbesondere eine Streckblasmaschine, welche die zugeführten Kunststoffvorformlinge zu Kunststoffbehältnissen umformt.

Besonders bevorzugt handelt es sich bei dieser Umformungseinrichtung um eine sterile Umformungseinrichtung, d.h. eine Umformungseinrichtung, welche einen Reinraum aufweist, innerhalb dessen die Kunststoffvorformlinge zu den Kunststoffbehältnissen umgeformt werden. Bevorzugt ist dieser Reinraum kanalartig um einen Transportpfad der Kunststoffvorformlinge ausgebildet.

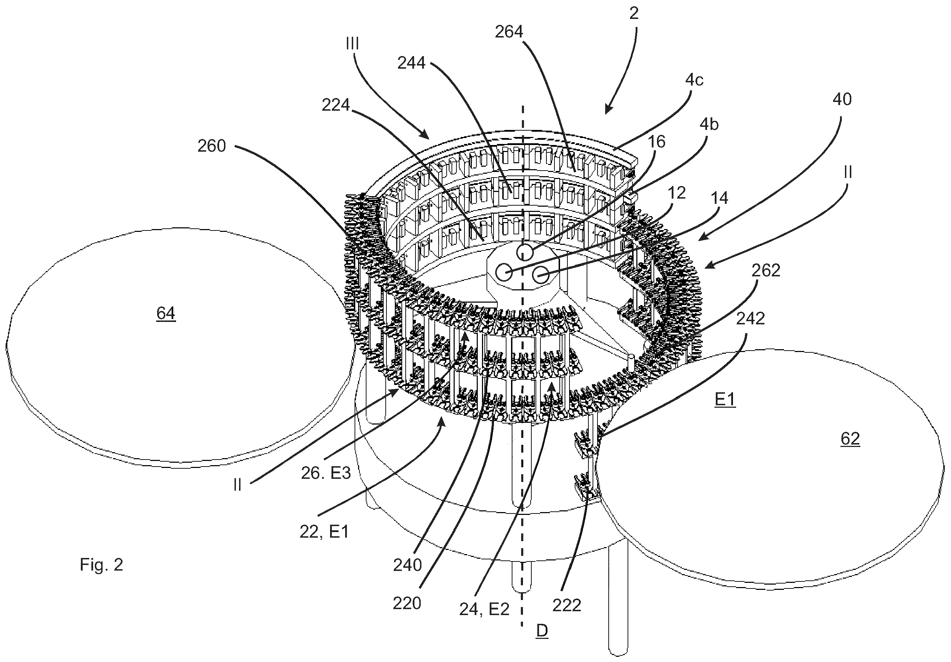

Fig. 2 zeigt eine Darstellung der erfindungsgemäßen Transporteinrichtung 2. Diese weist eine erste Transporteinheit 22 auf, welche in einer ersten Ebene E1 drehbar bezüglich einer Drehachse D angeordnet ist. Das Bezugszeichen 24 kennzeichnet eine zweite Transporteinheit, welche ebenfalls drehbar bezüglich der Drehachse D in einer zweiten Ebene E2 angeordnet ist, welche in Richtung der Drehachse D gegenüber der ersten Ebene E1 versetzt ist.

Das Bezugszeichen 26 kennzeichnet eine dritte Transporteinheit, welche in einer dritten Ebene E3 drehbar bezüglich einer Drehachse D angeordnet ist. Die dritte Ebene E3 ist dabei gegenüber der ersten Ebene E1 und der zweiten Ebene E2 in Richtung der Drehachse versetzt.

Bevorzugt sind die Drehbewegungen der einzelnen Transporteinheiten 22, 24, 26 bezüglich der Drehachse miteinander gekoppelt.

Die erste Transporteinheit 22 weist eine Vielzahl von ersten Halteeinrichtungen 220, 222 und 224 auf, die jeweils zum Halten von Kunststoffvorformlingen geeignet und bestimmt sind. Genauer handelt es sich hierbei um einen ersten Anteil 220 von ersten Halteeinrichtungen, einen zweiten Anteil 222 von ersten Halteeinrichtungen und einen dritten Anteil 224 von ersten Halteeinrichtungen. Diese jeweiligen Anteile können jeweils gemeinsam in Richtung der Drehachse D verschoben werden, jedoch relativ zu den jeweils anderen Anteilen.

Die zweite Transporteinheit 24 weist eine Vielzahl von zweiten Halteeinrichtungen 240, 242 und 244 auf, die jeweils zum Halten von Kunststoffvorformlingen geeignet und bestimmt sind. Genauer handelt es sich hierbei um einen ersten Anteil 240 von zweiten Halteeinrichtungen, einen zweiten Anteil 242 von zweiten Halteeinrichtungen und einen dritten Anteil 244 von zweiten Halteeinrichtungen. Auch diese jeweiligen Anteile von Halteeinrichtungen können jeweils gemeinsam in Richtung der Drehachse verschoben werden, jedoch relativ zu den jeweils anderen Anteilen.

Die dritte Transporteinheit 26 weist eine Vielzahl von dritten Halteeinrichtungen 260, 262 und 264 auf, die jeweils zum Halten von Kunststoffvorformlingen geeignet und bestimmt sind. Genauer handelt es sich hierbei um einen ersten Anteil 260 von dritten Halteeinrichtungen, einen zweiten Anteil 262 von dritten Halteeinrichtungen und einen dritten Anteil 264 von dritten Halteeinrichtungen. Auch diese jeweiligen Anteile von Halteeinrichtungen können jeweils gemeinsam in Richtung der Drehachse D verschoben werden, jedoch relativ zu den jeweils anderen Anteilen.

Das Bezugszeichen 12 kennzeichnet eine erste Hubeinrichtung. Diese dient dazu, um den ersten Anteil 220 der ersten Halteeinrichtungen, welcher an der ersten Transporteinheit 22 angeordnet ist, den ersten Anteil 240 der zweiten Halteeinrichtungen, welcher an der zweiten Transporteinheit 24 angeordnet ist und den ersten Anteil 260 der dritten Halteeinrichtungen, welcher an der dritten Transporteinheit 26 angeordnet ist in Richtung der Drehachse D zu verschieben, ihn also anzuheben oder abzusenken.

Das Bezugszeichen 14 kennzeichnet eine zweite Hubeinrichtung. Diese dient dazu, um den zweiten Anteil 222 der ersten Halteeinrichtungen, welcher an der ersten Transporteinheit 22 angeordnet ist, den zweiten Anteil 242 der zweiten Halteeinrichtungen, welcher an der zweiten Transporteinheit 24 angeordnet ist und den zweiten Anteil 262 der dritten Halteeinrichtungen, welcher an der dritten Transporteinheit 26 angeordnet ist in Richtung der Drehachse D zu verschieben, ihn also anzuheben oder abzusenken.

Bei der in Fig. 2 gezeigten Situation sind diese jeweiligen zweiten Anteile gegenüber den ersten Anteilen in Richtung der Drehachse D abgesenkt.

Das Bezugszeichen 16 kennzeichnet eine dritte Hubeinrichtung. Diese dient dazu, um den dritten Anteil 224 der ersten Halteeinrichtungen, welcher an der dritten Transporteinheit 26 angeordnet ist, den dritten Anteil 244 der zweiten Halteeinrichtungen, welcher an der dritten Transporteinheit 26 angeordnet ist und den dritten Anteil 264 der dritten Halteeinrichtungen, welcher an der dritten Transporteinheit 26 angeordnet ist in Richtung der Drehachse D zu verschieben, ihn also anzuheben oder abzusenken.

Dabei werden jeweils die ersten, zweiten und dritten Anteile gemeinsam in Richtung der Drehachse verschoben. Die Bezugszeichen I - III kennzeichnen jeweils Segmente von gemeinsam miteinander verschiebbaren Halteeinrichtungen.

Die Bezugszeichen 4b und 4c kennzeichnen zwei Beaufschlagungseinrichtungen, welche dazu dienen um die von den Halteeinrichtungen gehaltenen Kunststoffvorformlinge (nicht gezeigt) mit einem fließfähigen Sterilisationsmittel und/oder sterilisierender Strahlung zu beaufschlagen.

Bevorzugt sind hier jeder Transporteinheit 22, 24, 26 mehrere, hier jeweils drei Beaufschlagungseinrichtungen zugeordnet. Bevorzugt entspricht die Anzahl der Beaufschlagungseinrichtungen pro Transporteinheit der Anzahl der Segmente.

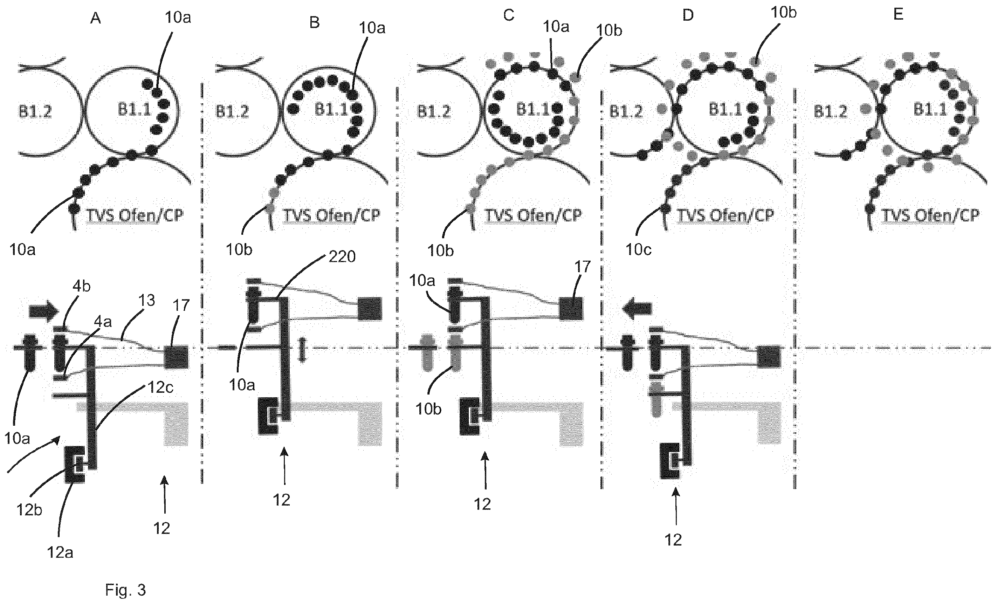

Fig. 3 veranschaulicht das erfindungsgemäße Verfahren. Dabei sind 5 Prozesssituationen A - E gezeigt, wobei sich die Prozesssituationen A - C auf einen Beladevorgang der Transporteinrichtung 2 mit Kunststoffvorformlingen 10a, 10b beziehen und die Prozesssituationen D und E auf einen Entladevorgang.

In der Prozesssituation A wird eine erste Gruppe an Kunststoffvorformlingen 10a der Transporteinrichtung zugeführt. Dabei werden diese Kunststoffvorformlinge jeweils von Halteeinrichtungen 220 des ersten Anteils der Halteeinrichtungen der ersten Transporteinheit gegriffen. Die Bezugszeichen 4a und 4b zeigen Beaufschlagungseinrichtungen, wobei die Beaufschlagungseinrichtungen jeweils den Halteeinrichtungen 220 zugeordnet sind.

In der Prozesssituation B werden die Halteeinrichtungen 220 von der Hubeinrichtung 12, welche hier eine Führungskurve 12a sowie eine Führungsrolle 12b und einen in der vertikalen Richtung V verschiebbaren Träger 12c aufweist, angehoben. Mit den Halteeinrichtungen werden auch die Beaufschlagungseinrichtungen 4a und 4b angehoben.

Bei der Prozesssituation C werden weitere Kunststoffvorformlinge 10b zugeführt, aber nunmehr dem nachfolgenden Segment, dessen Halteeinrichtungen noch nicht entlang der Drehachse verschoben wurden. Die genannten Halteeinrichtungen 220 verbleiben hier in der oberen Position. Man erkennt an dem oberen Teilbild der Prozesssituation dass nunmehr eine zweite Gruppe an Kunststoffvorformlingen 10b der Transporteinrichtung zugeführt wird.

Bei der Prozesssituation D werden die Halteeinrichtungen 220 wieder abgesenkt, so dass die ursprünglich zugeführten Kunststoffvorformlinge wieder von der Transporteinrichtung abgeführt werden können.

Bei der Prozesssituation E wurde zusätzlich eine dritte Gruppe an Kunststoffvorformlingen zugeführt.

Es wird darauf hingewiesen, dass sämtliche Merkmale, welche unter Bezugnahme auf das Verfahren beschrieben wurden, in entsprechender Weise auch für die Vorrichtung offenbart sind, was insbesondere bedeutet, dass die entsprechende Vorrichtung Einrichtungen aufweist, welche zur Durchführung der jeweiligen Verfahren geeignet und bestimmt sind. Weiterhin sind auch Merkmale, welche unter Bezugnahme auf die Vorrichtung beschrieben wurden entsprechend für das oder die Verfahren anwendbar. Dies bedeutet, dass die Verfahren unter Verwendung der entsprechenden Vorrichtungsmerkmale ausgeführt werden.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Vorrichtung (1) zum Behandeln und insbesondere Sterilisieren von Stückgütern und insbesondere von Kunststoffvorformlingen (10) oder Behältnissen mit einer Transporteinrichtung (2), welche dazu geeignet und bestimmt ist, die Stückgüter entlang eines kreisförmigen Transportpfads zu transportieren und mit wenigstens einer Beaufschlagungseinrichtung (4), welche dazu geeignet und bestimmt ist, die von der Transporteinrichtung transportierten Stückgüter zu sterilisieren, insbesondere mit Strahlung zu sterilisieren und/oder mit einem fließfähigen Reinigungs- und/oder Sterilisationsmittel zu beaufschlagen,
wobei die Transporteinrichtung (2) eine erste bezüglich einer Drehachse (D) drehbare Transporteinheit (22) aufweist, welche eine Vielzahl von ersten Halteeinrichtungen (220, 222, 224) aufweist, welche jeweils zum Halten der Stückgüter geeignet und bestimmt sind und welche äquidistant in einer bezüglich der Drehachse (D) senkrecht stehenden ersten Ebene (E1) angeordnet sind,
und wobei die Transporteinrichtung (2) eine zweite bezüglich der Drehachse (D) drehbare Transporteinheit (24) aufweist, welche eine Vielzahl von zweiten Halteeinrichtungen (240, 242, 244) aufweist, welche jeweils zum Halten der Stückgüter geeignet und bestimmt sind und welche äquidistant in einer bezüglich der Drehachse (D) senkrecht stehenden zweiten Ebene (E2) angeordnet sind, wobei die zweite Ebene (E2) in der Richtung der Drehachse gegenüber der ersten Ebene (E1)versetzt ist **dadurch gekennzeichnet, dass**
die Transporteinrichtung (2) wenigstens eine erste Hubeinrichtung (12) aufweist, welche dazu geeignet und bestimmt ist, einen vorgegebenen ersten Anteil der ersten Halteeinrichtungen (220) gegenüber einem vorgegebenen zweiten Anteil der ersten Halteeinrichtungen (222) in Richtung der Drehachse (D) zu verschieben.

2. Vorrichtung nach Anspruch 1,
**dadurch gekennzeichnet, dass**
die erste Hubeinrichtung dazu geeignet und bestimmt ist, einen vorgegebenen ersten Anteil der zweiten Halteeinrichtungen (240) gegenüber einem vorgegebenen zweiten Anteil der zweiten Halteeinrichtungen (242) in Richtung der Drehachse (D) zu verschieben, wobei bevorzugt diese Verschiebung des vorgegebenen ersten Anteils der zweiten Halteeinrichtungen (240) an die Verschiebung des vorgegebenen ersten Anteils der ersten Halteeinrichtungen (220) gekoppelt ist.

3. Vorrichtung nach wenigstens einem der vorangegangenen Ansprüche,
**dadurch gekennzeichnet, dass**
die Transporteinrichtung (2) einen Zuführbereich aufweist, in welchem der Transporteinrichtung die Stückgüter zuführbar sind, wobei die Stückgüter in einer Zuführebene zuführbar sind, bezüglich derer die Drehachse (D) senkrecht steht und die Transporteinrichtung (2) einen Abführbereich aufweist, in welchem die Stückgüter von der Transporteinrichtung abführbar sind, wobei die Stückgüter in einer Abführebene abführbar sind, bezüglich derer die Drehachse senkrecht steht, wobei bevorzugt die Zuführebene (Z) und die Abführebene (A) im Wesentlichen auf gleicher Höhe liegen.

4. Vorrichtung nach wenigstens einem der vorangegangenen Ansprüche,
**dadurch gekennzeichnet, dass**
die Transporteinrichtung (2) eine dritte bezüglich der Drehachse (D) drehbare Transporteinheit (26) aufweist, welche eine Vielzahl von dritten Halteeinrichtungen (260, 262, 264) aufweist, welche jeweils zum Halten der Stückgüter geeignet und bestimmt sind und welche äquidistant in einer bezüglich der Drehachse (D) senkrecht stehenden dritten Ebene (E3) angeordnet sind, wobei die dritte Ebene (E3) in der Richtung der Drehachse gegenüber der ersten Ebene (E1) und der zweiten Ebene (E2) versetzt ist und/oder die Transporteinrichtung (2) eine zweite Hubeinrichtung (14) aufweist, welche dazu geeignet und bestimmt ist, den vorgegebenen zweiten Anteil der ersten Halteeinrichtungen (226) gegenüber dem vorgegebenen ersten Anteil der ersten Halteeinrichtungen (222) in Richtung der Drehachse (D) zu verschieben.

5. Vorrichtung nach wenigstens einem der vorangegangenen Ansprüche,
**dadurch gekennzeichnet, dass**
die Transporteinrichtung (2) eine dritte Hubeinrichtung (16) aufweist, welche dazu geeignet und bestimmt ist, einen vorgegebenen dritten Anteil der ersten Halteeinrichtungen (226) gegenüber dem vorgegebenen ersten Anteil der ersten Halteeinrichtungen (222) und gegenüber dem vorgegebenen zweiten Anteil der ersten Halteeinrichtungen in Richtung der Drehachse (D) zu verschieben.

6. Vorrichtung nach wenigstens einem der vorangegangenen Ansprüche,
**dadurch gekennzeichnet, dass**
die zweite Hubeinrichtung dazu geeignet und bestimmt ist, einen vorgegebenen zweiten Anteil der zweiten Halteeinrichtungen (240) gegenüber einem vorgegebenen ersten Anteil der zweiten Halteeinrichtungen (242) in Richtung der Drehachse (D) zu verschieben, wobei bevorzugt diese Verschiebung des vorgegebenen zweiten Anteils der zweiten Halteeinrichtungen (240) an die Verschiebung des vorgegebenen zweiten Anteils der ersten Halteeinrichtungen (220) gekoppelt ist.

7. Vorrichtung nach wenigstens einem der vorangegangenen Ansprüche,
**dadurch gekennzeichnet, dass**
die Beaufschlagungseinrichtung eine Vielzahl von Beaufschlagungselementen und insbesondere Beaufschlagungsdüsen aufweist, welche jeweils den Halteeirichtungen zugeordnet sind und welche bevorzugt dazu geeignet und bestimmt ist, die Innenoberflächen und/oder die Außenoberflächen der Kunststoffvorformlinge zu beaufschlagen und/oder zu sterilisieren, insbesondere mit Strahlung zu sterilisieren.

8. Vorrichtung nach wenigstens einem der vorangegangenen Ansprüche,
**dadurch gekennzeichnet, dass**
die Vorrichtung eine Vielzahl von sich entlang einer kreissegmentförmigen Linie erstreckenden Trägersegmenten aufweist, welche jeweils zum gemeinsamen Tragen einer Vielzahl von Halteeinrichtungen geeignet und bestimmt ist, wobei bevorzugt jedes dieser Trägersegmente zum Tragen eines der Anteile der Halteeinrichtungen geeignet und bestimmt ist.

9. Vorrichtung nach wenigstens einem der vorangegangenen Ansprüche,
**dadurch gekennzeichnet, dass**
die Strahlung von wenigstens einer Strahlungserzeugungsvorrichtung, insbesondere Strahlungsquelle, als Elektronenstrahlung und/oder ultravioletter Strahlung bereitgestellt wird und/oder die Strahlung von der Beaufschlagungseinrichtung über eine, bevorzugt eine Vielzahl von Beaufschlagungselementen, insbesondere einen Strahlfinger, welcher insbesondere zur Innensterilisation in ein Inneres der Kunststoffvorformlinge eintaucht und insbesondere mit den Kunststoffvorformlingen mitbewegt wird, und/oder einen Flächenstrahler, welcher insbesondere zur Außensterilisation der Kunststoffvorformlinge geeignet und vorgesehen, insbesondere stationär relativ zur Transporteinrichtung angeordnet ist, aufgebracht wird.

10. Vorrichtung nach einem der vorhergehenden Ansprüche,
**dadurch gekennzeichnet, dass**
die Sterilisation als eine Außen- und/oder Innensterilisation, insbesondere auf derselben Transporteinrichtung ausführbar ist und/oder eine Außen- und/oder Innensterilisation auf wenigstens zwei, bevorzugt einer Vielzahl von, unterschiedlichen Transporteinrichtungen, insbesondere zeitlich und/oder örtlich nach- und/oder hintereinander, ausführbar sind.

11. Transporteinrichtung (2) zum Transportieren von Kunststoffvorformlingen oder Behältnissen entlang eines kreisförmigen Transportpfads
wobei die Transporteinrichtung (2) eine erste bezüglich einer Drehachse (D) drehbare Transporteinheit (22) aufweist, welche eine Vielzahl von ersten Halteeinrichtungen (220, 222, 224) aufweist, welche jeweils zum Halten der Kunststoffvorformlinge oder Behältnisse geeignet und bestimmt sind und welche äquidistant in einer bezüglich der Drehachse (D) senkrecht stehenden ersten Ebene (E1) angeordnet sind und wobei die Transporteinrichtung (2) eine zweite bezüglich der Drehachse (D) drehbare Transporteinheit (24) aufweist, welche eine Vielzahl von zweiten Halteeinrichtungen (240, 242, 244) aufweist, welche jeweils zum Halten der Kunststoffvorformlinge oder Behältnisse geeignet und bestimmt sind und welche äquidistant in einer bezüglich der Drehachse (D) senkrecht stehenden zweiten Ebene (E2) angeordnet sind, wobei die zweite Ebene (E2) in der Richtung der Drehachse gegenüber der ersten Ebene versetzt ist, wobei ein Versatz der zweiten Ebene gegenüber der ersten Ebene größer ist als eine Länge der zu transportierenden Kunststoffvorformlinge oder Behältnisse in der Richtung der Drehachse,
**dadurch gekennzeichnet, dass**
die Transporteinrichtung (2) wenigstens eine erste Hubeinrichtung (12) aufweist, welche dazu geeignet und bestimmt ist, einen vorgegebenen ersten Anteil der ersten Halteeinrichtungen (220) gegenüber einem vorgegebenen zweiten Anteil der ersten Halteeinrichtungen (222) in Richtung der Drehachse (D) zu verschieben und wobei die Transporteinrichtung (2) eine zweite Hubeinrichtung (14) aufweist, welche dazu geeignet und bestimmt ist, den vorgegebenen zweiten Anteil der ersten Halteeinrichtungen (226) gegenüber dem vorgegebenen ersten Anteil der ersten Halteeinrichtungen (222) in Richtung der Drehachse (D) zu verschieben.

12. Verfahren (1) zum Sterilisieren von Stückgütern und insbesondere von Kunststoffvorformlingen (10) oder Behältnissen wobei eine Transporteinrichtung (2), die Stückgüter entlang eines kreisförmigen Transportpfads transportiert und wenigstens eine Beaufschlagungseinrichtung (4) die von der Transporteinrichtung transportierten Stückgüter mit einem fließfähigen Sterilisationsmittel und/oder sterilisierender Strahlung beaufschlagt,
wobei die Transporteinrichtung (2) eine erste bezüglich einer Drehachse (D) drehbare Transporteinheit (22) aufweist, welche eine Vielzahl von ersten Halteeinrichtungen (220, 222, 224) aufweist, welche jeweils die Stückgüter halten und welche äquidistant in einer bezüglich der Drehachse (D) senkrecht stehenden ersten Ebene (E1) angeordnet sind,
und wobei die Transporteinrichtung (2) eine zweite bezüglich der Drehachse (D) drehbare Transporteinheit (24) aufweist, welche eine Vielzahl von zweiten Halteeinrichtungen (240, 242, 244) aufweist, welche jeweils die Stückgüter halten und welche äquidistant in einer bezüglich der Drehachse (D) senkrecht stehenden zweiten Ebene (E2) angeordnet sind, wobei die zweite Ebene (E2) in der Richtung der Drehachse gegenüber der ersten Ebene versetzt ist,
**dadurch gekennzeichnet, dass**
die Transporteinrichtung (2) wenigstens eine erste Hubeinrichtung (12) aufweist, welche wenigstens zeitweise einen vorgegebenen ersten Anteil der ersten Halteeinrichtungen (220) gegenüber einem vorgegebenen zweiten Anteil der ersten Halteeinrichtungen (222) in Richtung der Drehachse (D) verschiebt.

13. Verfahren nach dem vorangegangenen Anspruch,
**dadurch gekennzeichnet, dass**
die Kunststoffvorformlinge oder Kunststoffbehältnisse der Transporteinrichtung in einer Zuführebene (Z) zugeführt werden, zu welcher die Drehachse senkrecht steht und die Kunststoffvorformlinge oder Kunststoffbehältnisse von der Transporteinrichtung in einer Abführebene (A) abgeführt werden, zu welcher die Drehachse senkrecht steht und bevorzugt die Zuführebene (Z) und die Abführebene (A) im Wesentlichen zusammenfallen und/oder jeder Kunststoffvorformling und/oder jedes Kunststoffbehältnis um einen Transportwinkel transportiert wird, der größer ist als 360°, bevorzugt größer als 720°.

14. Verfahren nach wenigstens einem der vorangegangenen Ansprüche,
**dadurch gekennzeichnet, dass**
jeder Kunststoffvorformling wenigstens einmal in Richtung der Drehachse (D) in einer ersten Verschieberichtung verschoben wird und wenigstens einmal in Richtung der Drehachse in einer zweiten Verschieberichtung, welche der ersten Verschieberichtung entgegengesetzt ist verschoben wird und/oder jeder Kunststoffvorformling und/oder jeder Kunststoffvorformling wenigstens zweimal und bevorzugt wenigstens dreimal in Richtung der Drehachse in einer ersten Verschieberichtung verschoben wird.

15. Verfahren nach wenigstens einem der vorangegangenen Ansprüche,
**dadurch gekennzeichnet, dass**
Anteile von in der Transportrichtung hintereinander angeordneten Kunststoffvorformlinge und/oder Kunststoffbehältnisse gemeinsam entlang der Drehachse verschoben werden und/oder dass übereinander angeordnete Kunststoffvorformlinge oder Behältnisse gemeinsam entlang der Drehachse verschoben werden.
